# EUROPEAN PATENT APPLICATION

(11) **EP 4 682 789 A1**
(43) Date of publication of application: **21.01.2026**
(21) Application number: 23927536.5
(22) Date of filing: 16.03.2023
(51) Int. Cl.: G06Q 10/06

(54) **WORKFLOW GENERATION METHOD, INFORMATION PROCESSING DEVICE, AND WORKFLOW GENERATION PROGRAM**

(71) Applicant: FUJITSU LIMITED, Kawasaki-shi, Kanagawa 211-8588 (JP)
(72) Inventor: NAKAO, Manabu, Kawasaki-shi, Kanagawa 211-8588 (JP); INOMATA, Akihiro, Kawasaki-shi, Kanagawa 211-8588 (JP); SAWASAKI, Naoyuki, Kawasaki-shi, Kanagawa 211-8588 (JP); KURAKI, Kensuke, Kawasaki-shi, Kanagawa 211-8588 (JP)
(74) Representative: Hoffmann Eitle
(86) International application number: PCT/JP2023/010408
(87) International publication number: WO 2024/189917

(57) **Abstract**

A workflow generation method in which a computer executes processing of calculating, in a case where a conditional branch item included in a workflow is acquired, an indicator value in a case where any one of a plurality of candidate items is used by using a machine learning model, by referring to a storage that stores the plurality of candidate items satisfying a condition of a branch destination corresponding to the conditional branch item and the machine learning model that calculates the indicator value of each of the plurality of candidate items, selecting an item of which the calculated indicator value satisfies a predetermined condition from among the plurality of candidate items, and generating a workflow in which the selected item is arranged at the branch destination of the conditional branch item.

## Description

### [Technical Field]

The present embodiment relates to a workflow generation method, an information processing apparatus, and a workflow generation program.

### [Background Art]

In government authorities such as national and local governments, measure planning is sometimes carried out to provide health services and the like to residents.

For example, there is a technology of estimating an effect of implementing a measure based on a learning result obtained by vectorizing healthcare data and using a target achievement rate and a measure effect information actual measurement effect as objective variables.

### [Prior Art Reference]

### [Patent Document]

[Patent Document 1] JP 2021-89523 A

### [Summary of the Invention]

### [Problems to be Solved by the Invention]

However, in actual measure planning practices, a measure proposal is often based on intuition, experience, and assumptions, which may make it difficult to generate a workflow aimed at achieving a target measure indicator (key performance indicator: KPI).

In one aspect, an object is to improve workflow generation efficiency.

### [Means to Solve the Problem]

According to an aspect of embodiment(s), a workflow generation method in which a computer executes processing of calculating, in a case where a conditional branch item included in a workflow is acquired, an indicator value in a case where any one of a plurality of candidate items is used by using a machine learning model, by referring to a storage that stores the plurality of candidate items satisfying a condition of a branch destination corresponding to the conditional branch item and the machine learning model that calculates the indicator value of each of the plurality of candidate items, selecting an item of which the calculated indicator value satisfies a predetermined condition from among the plurality of candidate items, and generating a workflow in which the selected item is arranged at the branch destination of the conditional branch item.

### [Effect of Invention]

In one aspect, workflow generation efficiency can be improved.

### [Brief Description of Drawings]

Fig. 1 is a diagram for describing measure planning in a related example.
Fig. 2 is a diagram illustrating a prediction model as an embodiment.
Fig. 3 is a diagram for describing propagation of an influence between items in the prediction model as the embodiment.
Fig. 4 is a diagram for describing a service implementation item included in the prediction model as the embodiment.
Fig. 5 is a diagram for describing calculation of an overall effect and cost of a measure in the prediction model as the embodiment.
Fig. 6 is a diagram for describing replacement processing for the service implementation item in the prediction model as the embodiment.
Fig. 7 is a flowchart for describing the replacement processing for the service implementation item in the prediction model as the embodiment.
Fig. 8 is a block diagram schematically illustrating a hardware configuration example of an information processing apparatus in the embodiment.

### DESCRIPTION OF EMBODIMENT(S)

### [A] Related Example

In order to extend the healthy life expectancy of the population, the national government encourages local governments to develop data health plans and implement efficient health programs.

As the insurance business, there is a measure such as a follow-up measure for chronic kidney disease (CKD), in which a citizen identified as high-risk through medical examination receives medical consultations and health guidance from a specialist.

The citizen is advised to undergo medical consultation by a specialist, for example, based on a numerical value of a test value of a medical examination result as a determination criterion. For example, when a urinary protein level is 2+ or higher or an estimated glomerular filtration rate (eGFR) is below 60 ml/min/1.73 m2, the citizen is advised to undergo a test by a specialist.

In measure planning, when the numerical value of the test value serving as the determination criterion is changed, an influence of the measure may be changed.

There is a desire to loosen the determination criterion in order to increase the number of people referred to specialists. However, in a case where the determination criterion is excessively relaxed, the number of referred individuals may become too large for specialists to handle.

A target measure is expressed, for example, in the form of a workflow including a plurality of conditional branches and a plurality of service implementations. The plurality of conditional branches are sequentially followed based on state data of an individual as a measure target to determine "which service to allocate".

As an evaluation indicator for the measure, an effect, a cost, the number of people who receive a service, or the like may be used. The effect of the follow-up measure for CKD is evaluated in terms of, for example, how much the number of new chronic kidney disease patients (= the number of citizens × incidence rate) has decreased, the cost of the follow-up measure for CKD is evaluated in terms of a financial burden on the local government caused by implementation of medical examination or treatment provided by each doctor, and the number of people who receive a service is the number of people who receive the medical examination or a service provided by each doctor.

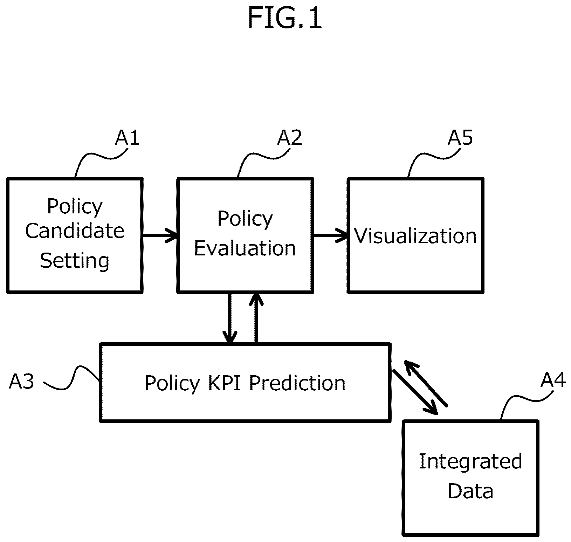

Fig. 1 is a diagram for describing measure planning in the related example.

There is a technology of predicting a key performance indicator (KPI) of a candidate measure from integrated data for each individual including various types, and supporting decision-making for measure planning.

In Fig. 1, in candidate measure setting denoted by reference sign A1, reducing "adult obesity prevalence" in a target region to 25% is set as a goal of a measure, providing exercise and nutrition guidance to younger local residents by physicians is set as the candidate measure, and the adult obesity prevalence within the region is set as the KPI.

Measure evaluation is performed on the set candidate measure as indicated by reference sign A2.

Measure KPI prediction is performed as indicated by reference sign A3. For example, a probability (classified into three levels of high, medium, and low) that each individual in the target region becomes obese within three years is predicted in a case where the candidate measure is executed.

The integrated data for the measure KPI prediction is referred to as indicated by reference sign A4. The integrated data may include, for example, a history (such as step count) of daily health information, a history of a diagnosis result, a history of a lifestyle, and a history of a test result for each local resident.

The measure evaluation is visualized as indicated by reference sign A5.

Therefore, a probability of becoming obese is calculated for each individual, and the obesity prevalence within the region is calculated.

However, a calculation time for effect prediction when the measure is changed may be increased. In the case of performing prediction using data of hundreds of thousands of citizens in order to calculate the probability of becoming obese for each individual, the result is not immediately output, and the output may take several to tens of minutes.

At the stage of considering measure modifications, a planner of the measure may desire to consider a useful measure modification by testing influences of changes to a certain part of the measure in various ways. It may be desired to shorten the calculation time to enable rapid iterations of evaluating modifications.

### [B] Embodiment

Hereinafter, an embodiment will be described with reference to the drawings. However, the embodiment described below is merely an example, and there is no intention to exclude the application of various modifications and technologies that are not explicitly described in the embodiment. That is, the present embodiment can be variously modified and implemented without departing from the gist thereof. Each drawing is not intended to include only the components illustrated in the drawing but may include other functions and the like. Hereinafter, the same reference signs in the respective drawings have similar functions, and thus the description thereof may be omitted.

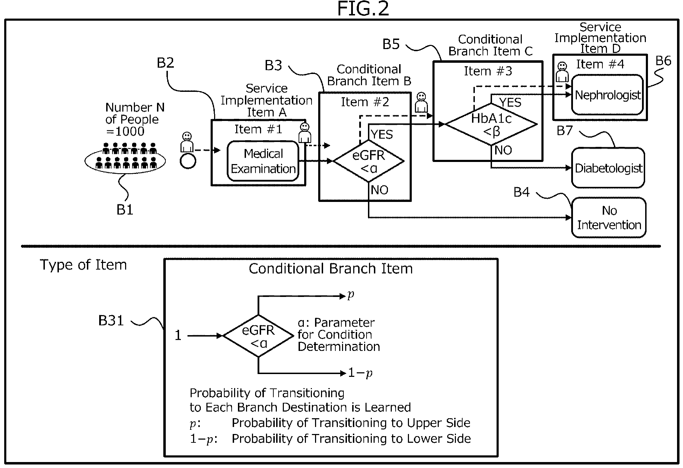

Fig. 2 is a diagram illustrating a prediction model as the embodiment.

A measure is modeled as a workflow including a combination of conditional branch items and service implementation items, for example. Then, the number of people who receive each service is predicted using a model trained by accumulating information of a flow of people from actual values used in the past for each conditional branch item and parameters.

In the example illustrated in Fig. 2, the number N of people = 1000 is input as indicated by reference sign B1.

An item #1 as a service implementation item A is set to "medical examination" as indicated by reference sign B2.

An item #2 as a conditional branch item B is set to "eGFR < α" as indicated by reference sign B3.

In the conditional branch item, a probability of transitioning to each branch destination is learned as indicated by reference sign B31. α is set as a parameter for condition determination, a probability of transitioning to an upper side of the conditional branch is p, and a probability of transitioning to a lower side of the conditional branch is 1-p.

In a case where "eGFR < α" is not satisfied (see NO branch of reference sign B3), it is determined that "no intervention" by a specialist is to be made for the citizen as indicated by reference sign B4.

On the other hand, in a case where "eGFR < α" is satisfied (see YES branch of reference sign B3), an item #3 as a conditional branch item C is set to "HbAlc < β" as indicated by reference sign B5.

In a case where "HbAlc < β" is satisfied (see YES branch of reference sign B5), an item #4 as a conditional branch item D is set as a "nephrologist" as indicated by reference sign B6, and it is determined that an intervention of the "nephrologist" is needed for the citizen.

On the other hand, in a case where "HbAlc < β" is not satisfied (see NO branch of reference sign B5), it is determined that an intervention of a "diabetologist" is needed for the citizen as indicated by reference sign B7.

In the example illustrated in Fig. 2, the number of people flowing through the item #1, the item #2, the item #3, and the item #4 in this order as indicated by dotted arrows is predicted.

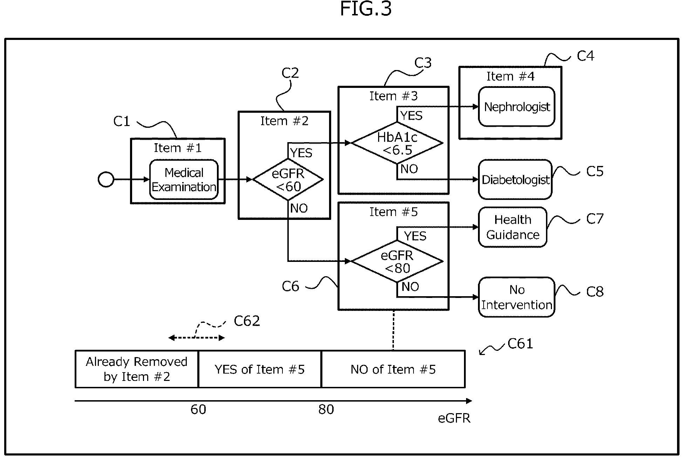

Fig. 3 is a diagram for describing propagation of an influence between items in the prediction model as the embodiment.

Downstream items may be affected by upstream items. Downstream items are affected by items used upstream and a parameter value of each item.

"Medical examination" is set as an item #1 as indicated by reference sign C1.

"eGFR < 60" is set as an item #2 as indicated by reference sign C2.

In a case where "eGFR < 60" is satisfied (see YES branch of reference sign C2), "HbAlc < 6.5" is set as an item #3 as indicated by reference sign C3.

In a case where "HbAlc < 6.5" is satisfied (see YES branch of reference sign C3), "nephrologist" is set as an item #4 as indicated by reference sign C4, and it is determined that an intervention of a "nephrologist" is needed for the citizen.

On the other hand, in a case where "HbAlc < 6.5" is not satisfied (see NO branch of reference sign C3), it is determined that an intervention of a "diabetologist" is needed for the citizen as indicated by reference sign C5.

In a case where "eGFR < 60" is not satisfied in reference sign C2 (see NO branch of reference sign C2), "eGFR < 80" is set as an item #5 as indicated by reference sign C6.

In a case where "eGFR < 80" is satisfied (see YES branch of reference sign C6), it is determined that "health guidance" is needed for the citizen as indicated by reference sign C7.

On the other hand, in a case where "eGFR < 80" is not satisfied (see NO branch of reference sign C6), it is determined that "no intervention" is to be made for the citizen as indicated by reference sign C8.

As indicated by reference sign C61, a proportion of people flowing to the YES branch and the NO branch of the item #5 is affected by a distribution of the people in the item #2.

As indicated by reference sign C62, when a determination threshold (60) for a parameter of the item #2 is changed, a range of people corresponding to the YES branch of the item #5 varies.

Therefore, the number of people who receive each service may be predicted using a model trained by accumulating a combination of items at the time of use in addition to information of a flow of people from actual values used in the past for each item and parameters.

In other words, the information of the flow of people may be learned from the actual values used in the past. Since the information of the flow of people varies depending on the combination of items and the parameters at the time of use, the information may be accumulated together so that the information of the flow of people can be predicted according to the combination of items and the parameters at the time of use.

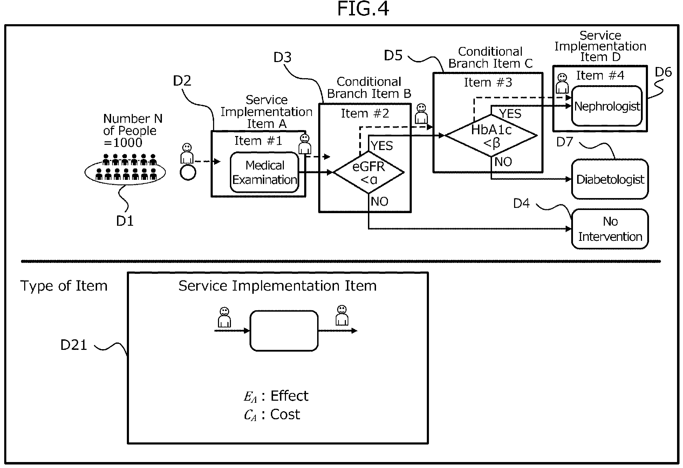

Fig. 4 is a diagram illustrating a service implementation item included in the prediction model as the embodiment.

Items D1 to D7 in Fig. 4 are similar to the items B1 to B7 in Fig. 2.

An influence (for example, the cost and effect) may be predicted using a model trained by accumulating information of an influence (for example, the cost and effect) from actual values used in the past for each item, a combination of items at the time of use, and parameters.

As indicated by reference sign D21, an effect EA and a cost CA when a service is implemented for one person may be learned for each service implementation item.

Since the effect EA and the cost CA vary depending on the combination of items and the parameters at the time of use, the effect EA and the cost CA may be accumulated together, so that the effect EA and the cost CA can be predicted according to the combination of items and the parameters at the time of use.

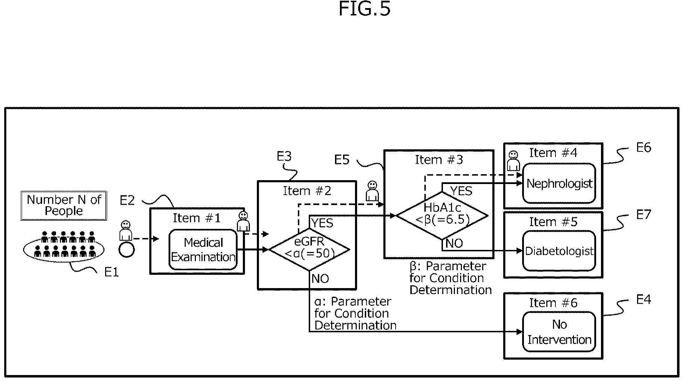

Fig. 5 is a diagram illustrating calculation of an overall effect and cost of the measure in the prediction model as the embodiment.

A regression model of service implementation items #4, #5, and #6 is used to determine the effect and cost per person.

In the example illustrated in Fig. 5, the number N of people is input as indicated by reference sign E1.

An item #1 is set to "medical examination" as indicated by E2, and the number of people flowing to the item #1 and the total effect are calculated. Assuming that the effect per person is EA = fA_E and the cost per person is CA = fA_C, when the number of people = N, the total effect = N × EA, and the total cost = N × CA.

An item #2 is set to "eGFR < α (= 50)" as indicated by reference sign E3, and the number of people = N is input. A branch probability pB of flowing to the YES branch of reference sign E3 when the parameter α = 50 is used in the order of the item #1 followed by the item #2 (item #1 → item #2) is pB = fB (α = 50) when a prediction model fB(α) trained by the item #2 is used, and the number of people = N × pB.

In a case where "eGFR < α" is not satisfied (see NO branch of reference sign E3), the item #6 is set to "no intervention" as indicated by reference sign E4, and it is determined that "no intervention" by a specialist is to be made for the citizen.

On the other hand, in a case where "eGFR < α" is satisfied (see YES branch of reference sign E3), an item #3 is set to "HbA1c < β (= 6.5)" as indicated by reference sign E5. when the parameter α = 50 and the parameter β = 6.5 are used in the order of the item #1 followed by the item #2 and the item #3 (item #1→ item #2→ item #3), a branch probability pC of flowing to the YES branch of reference sign E5 is pC = fC(α, β) when a prediction model fC (α = 50, β = 6.5) trained by the item #2 is used, and the number of people = N × pB × pC.

In a case where "HbA1c < β" is satisfied (see YES branch of reference sign E5), the item #4 is set as a "nephrologist" as indicated by reference sign E6, and it is determined that an intervention of the "nephrologist" is needed for the citizen. In addition, the number of people flowing to the item #4 and the total effect are calculated. Assuming that the effect per person is EA = fE_E and the cost per person is CA = fE_C, the number of people is Np = N × pA × pB, the total effect = Np × Ep, and the total cost = Np × CD. The number of people and the total effect are similarly calculated also for the items #5 and #6.

On the other hand, in a case where "HbA1c < β" is not satisfied (see NO branch of reference sign E5), the item #5 is set as a "diabetologist" as indicated by reference sign E7, and it is determined that an intervention of the "diabetologist" is needed for the citizen.

The overall effect of the measure = the total effect on the people flowing to the item #1 + the total effect on the people flowing to the item #4 + the total effect on the people flowing to the item #5 + the total effect on the people flowing to the item #6 The overall cost of the measure = the total cost for the people flowing to the item #1 + the total cost for the people flowing to the item #4 + the total cost for the people flowing to the item #5 + the total cost for the people flowing to the item #6.

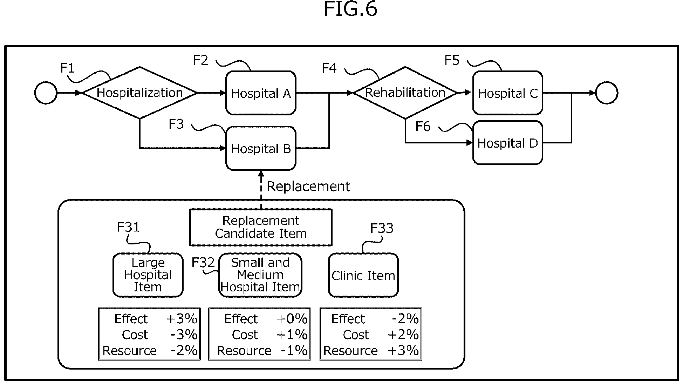

Fig. 6 is a diagram for describing replacement processing for the service implementation item in the prediction model as the embodiment.

In the prediction model illustrated in Fig. 6, service implementation items "hospital A" (see reference sign F2) and "hospital B" (see reference sign F3) are arranged downstream of a conditional branch item "hospitalization" (see reference sign F1). Furthermore, in the prediction model illustrated in Fig. 6, a conditional branch item "rehabilitation" (see reference sign F4) is arranged downstream of the service implementation items "hospital A" and "hospital B", and service implementation items "hospital C" (see reference sign F5) and "hospital D" (see reference sign F6) are arranged downstream of the conditional branch item "rehabilitation".

In the example illustrated in Fig 6, the service implementation item "hospital B" is replaced with a replacement candidate item. The replacement candidate item may include, for example, a "large hospital item" (see reference sign F31), a "small and medium hospital item" (see reference sign F32), and a "clinic item" (see reference sign F33).

Each of the replacement candidate items has indicator values such as an effect, a cost, and a resource. The effect indicates an increase ratio of a cure rate or a prevention rate for a disease when the replacement candidate item is used. The cost indicates a reduction ratio in expenses incurred when the replacement candidate item is used. The resource indicates a reduction ratio in human resources and material resources when the replacement candidate item is used.

In the example illustrated in Fig 6, in the case of the replacement candidate item "large hospital item", the effect is +3%, the cost is -3%, and the resource is -2%. In the case of the replacement candidate item "small and medium hospital item", the effect is +0%, the cost is +1%, and the resource is -1%. In the case of the replacement candidate item "clinic item" the effect is -2%, the cost is +2%, and the resource is +3%.

When the indicator value (any one or all of the effect, the cost, and the resource) of the service implementation item "hospital B" is less than a threshold in the prediction model illustrated in Fig. 6, the service implementation item "hospital B" may be replaced with the replacement candidate item. The indicator value may be a total value obtained by weighting and adding the values of the effect, the cost, and the resource.

In the example illustrated in Fig. 6, only the service implementation item "hospital B" is replaced with the replacement candidate item. However, other service implementation items "hospital A", "hospital C", and "hospital D" included in the prediction model may also be replaced with the replacement candidate items.

In addition, whether or not the indicator value satisfies the condition may be determined for each group (in other words, conditional branch group) of the conditional branch item and the service implementation items arranged immediately after the conditional branch item. In the example illustrated in Fig. 6, the conditional branch groups include a group including the conditional branch group "hospitalization" and the service implementation items "hospital A" and "hospital B", and a group including the conditional branch group "rehabilitation" and the service implementation items "hospital C" and "hospital D". In a case where there is a service implementation item of which the indicator value does not satisfy the condition in the conditional branch group, replacement with the replacement candidate item may be performed for all the service implementation items in the conditional branch group.

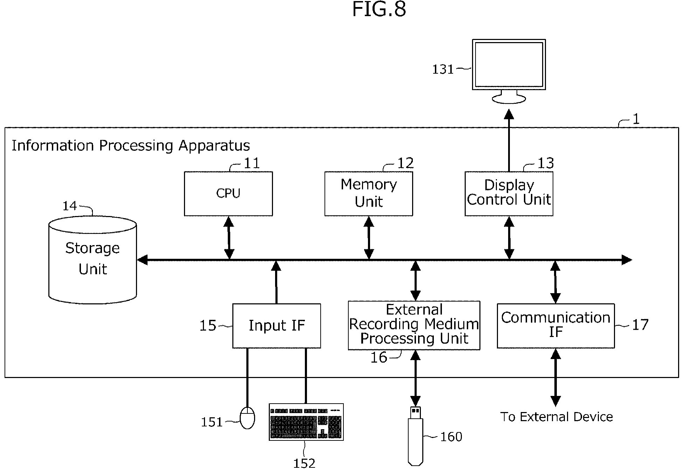

In other words, an information processing apparatus 1 (described below with reference to Fig. 8) calculates, in a case where a conditional branch item included in a workflow is acquired, an indicator value in a case where any one of a plurality of candidate items is used by using a machine learning model that calculates the indicator value of each of the plurality of candidate items by referring to a storage 14 (described below with reference to Fig. 8) that stores the plurality of candidate items satisfying a condition of a branch destination corresponding to the conditional branch item and the machine learning model. The information processing apparatus 1 selects an item of which the calculated indicator value satisfies a predetermined condition from among the plurality of candidate items. The information processing apparatus 1 generates a workflow in which the selected item is arranged at the branch destination of the conditional branch item.

In addition, in a case where another workflow in which some of the plurality of candidate items are arranged is acquired, the information processing apparatus 1 may extract some candidate items from the acquired another workflow, and train the machine learning model by using the indicator values regarding the some candidate items when the another workflow is executed.

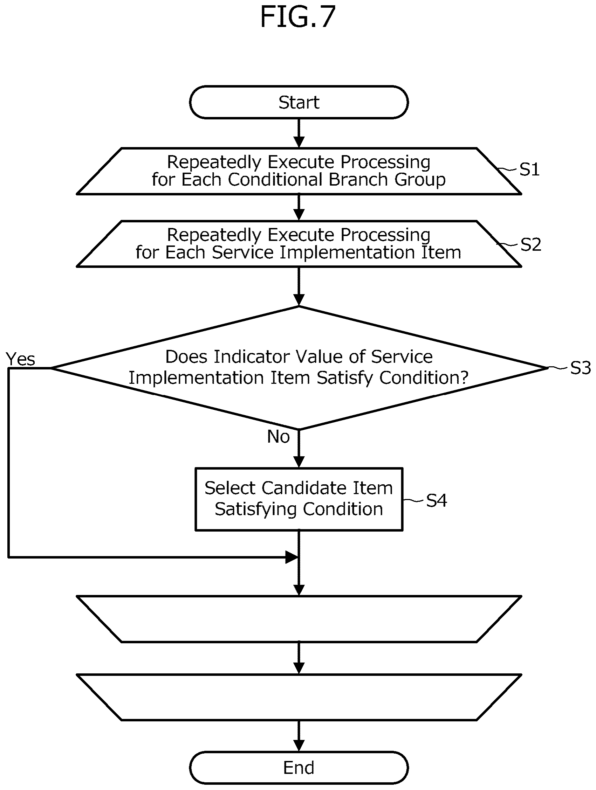

Processing of replacing the service implementation item in the prediction model as the embodiment will be described with reference to the flowchart (steps S1 to S4) illustrated in Fig. 7.

The information processing apparatus 1 (described below with reference to Fig. 8) repeatedly executes the processing of the following steps S2 to S4 for each conditional branch group (step S1). Note that the repetitive processing in step S1 may be omitted.

The information processing apparatus 1 repeatedly executes the following processing of steps S3 and S4 for each of the service implementation items (step S2). Note that the repetitive processing in step S2 may be omitted.

The information processing apparatus 1 determines whether or not the indicator value of the service implementation item satisfies the condition (step S3).

In a case where the indicator value of the service implementation item satisfies the condition (see Yes branch in step S3), the next repetition in steps S1 and S2 is performed, or the replacement processing for the service implementation item in the prediction model ends.

On the other hand, in a case where the indicator value of the service implementation item does not satisfy the condition (see No branch in step S3), the information processing apparatus 1 selects a replacement candidate item that satisfies the condition (step S4). Then, the next repetition in steps S1 and S2 is performed, or the replacement processing for the service implementation item in the prediction model ends.

In a case where there is no replacement candidate item that satisfies the condition, priority may be assigned to each of the effect, the cost, and the resource in the indicator value, and a replacement candidate item that satisfies the condition for the indicator value with the highest priority may be selected.

Fig. 8 is a block diagram schematically illustrating a hardware configuration example of the information processing apparatus 1 in the embodiment.

As illustrated in Fig. 8, the information processing apparatus 1 includes a central processing unit (CPU) 11, a memory unit 12, a display control unit 13, a storage 14, an input interface (IF) 15, an external recording medium processing unit 16, and a communication IF 17.

The memory unit 12 is an example of a storage, and is, for example, a read only memory (ROM), a random access memory (RAM), and the like. A program such as Basic Input/Output System (BIOS) may be written in the ROM of the memory unit 12. A software program of the memory unit 12 may be appropriately read and executed by the CPU 11. In addition, the RAM of the memory unit 12 may be used as a temporary recording memory or a working memory.

The display control unit 13 is connected to a display device 131 and controls the display device 131. The display device 131 is a liquid crystal display, an organic light-emitting diode (OLED) display, a cathode ray tube (CRT), an electronic paper display, or the like, and displays various types of information to an operator or the like. The display device 131 may be combined with an input device and may be, for example, a touch panel.

As the storage 14, for example, a dynamic random access memory (DRAM), a solid-state drive (SSD), a storage class memory (SCM), or a hard disk drive (HDD) may be used.

The input IF 15 may be connected to an input device such as a mouse 151 or a keyboard 152 and control the input device such as the mouse 151 or the keyboard 152. The mouse 151 and the keyboard 152 are examples of the input devices, and the operator performs various input operations via these input devices.

The external recording medium processing unit 16 is configured such that a recording medium 160 can be mounted. The external recording medium processing unit 16 is configured to be able to read information recorded on the recording medium 160 in a state in which the recording medium 160 is mounted. In this example, the recording medium 160 is portable. For example, the recording medium 160 is a flexible disk, an optical disk, a magnetic disk, a magneto-optical disk, a semiconductor memory, or the like.

The communication IF 17 is an interface that enables communication with an external device.

The CPU 11 is an example of a processor (in other words, a computer), and is a processing device that performs various controls and computations. The CPU 11 implements various functions by executing an operating system (OS) and a program read into the memory unit 12. Note that the CPU 11 may be a multiprocessor including a plurality of CPUs, a multi-core processor including a plurality of CPU cores, or a configuration including a plurality of multi-core processors.

The device that controls the overall operation of the information processing apparatus 1 is not limited to the CPU 11 and may be, for example, any one of an MPU, a DSP, an ASIC, a PLD, and an FPGA. Furthermore, the device that controls the overall operation of the information processing apparatus 1 may be a combination of two or more of the CPU, the MPU, the DSP, the ASIC, the PLD, and the FPGA. Note that the MPU is an abbreviation for micro processing unit, DSP is an abbreviation for digital signal processor, and ASIC is an abbreviation for application specific integrated circuit. In addition, PLD is an abbreviation for programmable logic device, and FPGA is an abbreviation for field programmable gate array.

### [C] Effects

According to the workflow generation method, the information processing apparatus, and the workflow generation program in the above-described embodiment, for example, the following operational effects can be achieved.

The information processing apparatus 1 calculates, in a case where a conditional branch item included in a workflow is acquired, an indicator value in a case where any one of a plurality of candidate items is used by using a machine learning model that calculates the indicator value of each of the plurality of candidate items by referring to the storage 14 that stores the plurality of candidate items satisfying a condition of a branch destination corresponding to the conditional branch item and the machine learning model. The information processing apparatus 1 selects an item of which the calculated indicator value satisfies a predetermined condition from among the plurality of candidate items. The information processing apparatus 1 generates a workflow in which the selected item is arranged at the branch destination of the conditional branch item.

As a result, workflow generation efficiency can be improved.

In a case where another workflow in which some of the plurality of candidate items are arranged is acquired, the information processing apparatus 1 extracts some candidate items from the acquired another workflow, and trains the machine learning model by using the indicator values regarding the some candidate items when the another workflow is executed.

As a result, it is possible to efficiently train the learning model.

The indicator value includes a value representing at least one of the effect, the cost, and the resource when each of the plurality of candidate items is used.

As a result, a candidate item suitable for the workflow can be arranged.

### [D] Others

The disclosed technology is not limited to the above-described embodiment, and various modifications can be made without departing from the gist of the present embodiment. Each configuration and each processing of the present embodiment can be selected or omitted as needed or may be appropriately combined.

In the above-described embodiment, the prediction model for a workflow in a health service provided by government authorities is generated, but the disclosed technology is not limited thereto. The above-described embodiment may be used for generating prediction models for various workflows such as work, tests, and questionnaires having conditional branches. Also in this case, the same operational effects as those of the above-described embodiment can be obtained.

### [Reference Signs List]

1 Information processing apparatus
11 CPU
12 Memory unit
13 Display control unit
14 Storage device
16 External recording medium processing unit
131 Display device
151 Mouse
152 Keyboard
160 Recording medium
15 Input IF
17 Communication IF

## Claims

1. A workflow generation method in which a computer executes processing of:
calculating, in a case where a conditional branch item included in a workflow is acquired, an indicator value in a case where any one of a plurality of candidate items is used by using a machine learning model, by referring to a storage that stores the plurality of candidate items satisfying a condition of a branch destination corresponding to the conditional branch item and the machine learning model that calculates the indicator value of each of the plurality of candidate items;
selecting an item of which the calculated indicator value satisfies a predetermined condition from among the plurality of candidate items; and
generating a workflow in which the selected item is arranged at the branch destination of the conditional branch item.

2. The workflow generation method according to claim 1, wherein the computer executes processing of
extracting, in a case where another workflow in which some of the plurality of candidate items are arranged is acquired, some candidate items from the acquired another workflow, and training the machine learning model by using the indicator values regarding the some candidate items when the another workflow is executed.

3. The workflow generation method according to claim 1 or 2, wherein
the indicator value includes a value representing at least one of an effect, a cost, and a resource when each of the plurality of candidate items is used.

4. An information processing apparatus comprising a processor configured to:
calculate, in a case where a conditional branch item included in a workflow is acquired, an indicator value in a case where any one of a plurality of candidate items is used by using a machine learning model, by referring to a storage that stores the plurality of candidate items satisfying a condition of a branch destination corresponding to the conditional branch item and the machine learning model that calculates the indicator value of each of the plurality of candidate items;
select an item of which the calculated indicator value satisfies a predetermined condition from among the plurality of candidate items; and
generate a workflow in which the selected item is arranged at the branch destination of the conditional branch item.

5. The information processing apparatus according to claim 4, wherein
in a case where another workflow in which some of the plurality of candidate items are arranged is acquired, the processor extracts some candidate items from the acquired another workflow, and trains the machine learning model by using the indicator values regarding the some candidate items when the another workflow is executed.

6. The information processing apparatus according to claim 4 or 5, wherein
the indicator value includes a value representing at least one of an effect, a cost, and a resource when each of the plurality of candidate items is used.

7. A workflow generation program causing a computer to execute processing of:
calculating, in a case where a conditional branch item included in a workflow is acquired, an indicator value in a case where any one of a plurality of candidate items is used by using a machine learning model, by referring to a storage that stores the plurality of candidate items satisfying a condition of a branch destination corresponding to the conditional branch item and the machine learning model that calculates the indicator value of each of the plurality of candidate items;
selecting an item of which the calculated indicator value satisfies a predetermined condition from among the plurality of candidate items; and
generating a workflow in which the selected item is arranged at the branch destination of the conditional branch item.

8. The workflow generation program according to claim 7, wherein the computer is caused to execute processing of
extracting, in a case where another workflow in which some of the plurality of candidate items are arranged is acquired, some candidate items from the acquired another workflow, and training the machine learning model by using the indicator values regarding the some candidate items when the another workflow is executed.

9. The workflow generation program according to claim 7 or 8, wherein
the indicator value includes a value representing at least one of an effect, a cost, and a resource when each of the plurality of candidate items is used.
